# EUROPEAN PATENT APPLICATION

(11) **EP 2 762 112 A1**
(43) Date of publication of application: **06.08.2014**
(21) Application number: 12194420.1
(22) Date of filing: 27.11.2012
(51) Int. Cl.: A61F 13/00

(54) **Hydrogel laminate, method for making the same, and wound dressing.**

(30) Priority: 29.11.2011 TW 100143715
(71) Applicant: KANG NA HSIUNG ENTERPRISE CO. LTD., 72256 Tainan (TW)
(72) Inventor: Hu, Yen-Jung, 72256 TAINAN CITY (TW); Keng, Chun-Lan, 80261 KAOHSIUNG CITY (TW)
(74) Representative: Regimbeau

(57) **Abstract**

A method for manufacturing a hydrogel laminate (10) of a wound dressing (100) includes the steps of: providing a fabric layer (2); forming a fluid-impermeable barrier layer (1) on a lower surface of the fabric layer (2), the fluid-impermeable barrier layer (1) having a water-repellant surface bonded to the lower surface of the fabric layer (2); applying a hydrogel precursor (3) onto an upper surface of the fabric layer (2) so as to permit the hydrogel precursor (3) to infiltrate into the fabric layer (2); and curing the hydrogel precursor (3) in the fabric layer (2) to obtain a hydrogel laminate (10).

## Description

This invention relates to a method for manufacturing a hydrogel laminate and use of the hydrogel laminate. Specifically, the hydrogel laminate can be used as a component of a wound dressing.

A hydrogel wound dressing is commonly used in the treatment and management of burns and ulcerated wounds, and a hydrogel layer of the hydrogel wound dressing provides moisture at a wound/dressing interface to avoid tearing or ripping of the wound when the hydrogel wound dressing is removed. However, delamination has been a persistent and major problem when the hydrogel wound dressing is applied to heavily exudating wounds.

US Patent No. 6, 468, 383 discloses a method for making a hydrogel laminate which includes an adhesive polymer-coated substrate and a hydrogel made from hydrophilic polymer. In this method, electric beam radiation allows the copolymerization between an adhesive polymer of the adhesive polymer-coated substrate and the hydrophilic polymer, and the crosslinking of the hydrophilic polymer for the formation of a hydrogel. The hydrogel laminate manufactured using such method has improved delamination resistance. However, the adhesive layer used in the hydrogel laminate nonetheless undergoes adhesive failure due to the exposure to fluid. Moreover, such method for preparation of the hydrogel laminate requires the use of a mold during the manufacturing process. This is inefficient and costly. There is a long felt need for a method to prepare a hydrogel laminate that is highly resistant to delamination yet manufactured in a continuous process with high yield and productivity.

Therefore, the object of the present invention is to provide a hydrogel laminate for a wound dressing that is resistant to delamination and a method for making the same.

According to a first aspect, the present invention provides a method for manufacturing a hydrogel laminate of a wound dressing, comprising the steps of:
(a) providing a fabric layer;
(b) forming a fluid-impermeable barrier layer on a lower surface of the fabric layer, the fluid-impermeable barrier layer having a water-repellant surface bonded to the lower surface of the fabric layer;
(c) applying a hydrogel precursor onto an upper surface of the fabric layer so as to permit the hydrogel precursor to infiltrate into the fabric layer; and
(d) curing the hydrogel precursor in the fabric layer to obtain a hydrogel laminate.

In a second aspect of the present invention, there is provided a hydrogel laminate for a wound dressing, comprising:
a fabric layer;
a fluid-impermeable barrier layer having a water-repellant surface bonded to a lower surface of said fabric layer; and
a cured hydrogel in the fabric layer.

In a third aspect of the present invention, there is provided a wound dressing comprising:
a vapor permeable film;
the aforesaid hydrogel laminate;
an adhesive layer disposed between the vapor permeable film and the hydrogel laminate and partially covered by the hydrogel laminate; and
a release film covering the hydrogel laminate and adhered to the adhesive layer which is exposed from the hydrogel laminate.

Other features and advantages of the present invention will become apparent in the following detailed description of the preferred embodiments of the invention, with reference to the accompanying drawings, in which:
Fig. 1 is a fragmentary schematic flow diagram showing consecutive steps of the preferred embodiment of a method for manufacturing a hydrogel laminate of this invention; and
Fig. 2 is a cross sectional view showing the preferred embodiment of a wound dressing of this invention that includes a hydrogel laminate.

Before the present invention is described in greater detail, it should be noted that same reference numerals have been used to denote like elements throughout the specification.

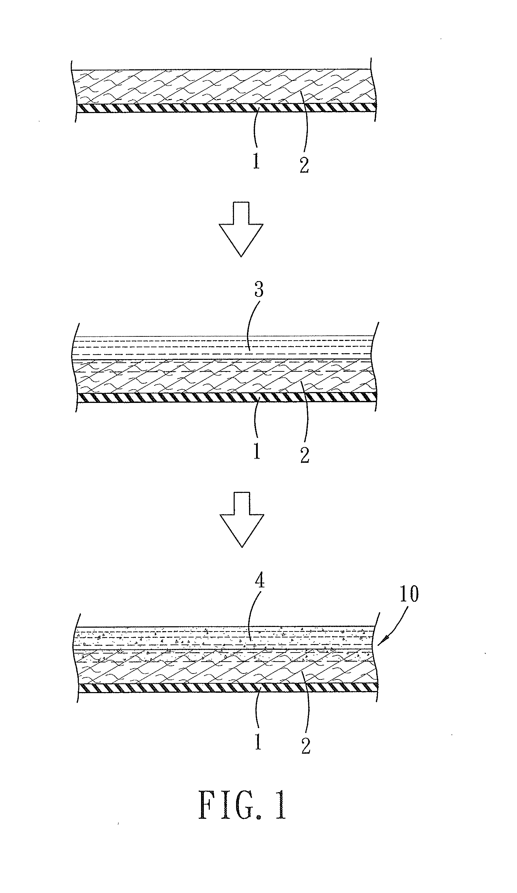

Referring to Figure 1, the preferred embodiment of a method for manufacturing a hydrogel laminate 10 of this invention comprises the following steps.

A film composite including a fluid-impermeable barrier layer 1 and a fabric layer 2 is provided. The fluid-impermeable barrier layer 1 has a water-repellant surface bonded to a lower surface of the fabric layer 2. The fluid-impermeable barrier layer 1 can be formed by extrusion coating a molten thermoplastic material on the fabric layer 2, followed by cooling. Under such method, the fluid-impermeable barrier layer 1 is preferably made of a material that has a melting point lower than that of the fabric layer 2. Examples of the thermoplastic material for the fluid-impermeable barrier layer 1 include polyethylene, polypropylene, thermoplastic polyurethane, and combinations thereof. The fluid-impermeable barrier layer 1 can also be laminated onto the lower surface of the fabric layer 2. Lamination is implemented by heating or pressing a film of a plastic material upon the fabric layer 2 so as to permit the film to be laminated with the fabric layer 2. Examples of the plastic material for the fluid-impermeable barrier layer 1 include polyethylene, polypropylene, thermoplastic polyurethane, and combinations thereof. The fabric layer 2 can be nonwoven, woven or knitted fabric. Preferably, the fabric layer 2 is a nonwoven fabric. Preferably, the nonwoven fabric can be extrusion coated nonwoven or film laminated nonwoven fabric.

Next, a hydrogel precursor 3 is applied onto an upper surface of the fabric layer 2 to permit infiltration of the hydrogel precursor 3 into the fabric layer 2. The fluid-impermeable barrier layer 1 that is affixed to the lower surface of the fabric layer 2 can prevent the uncured hydrogel precursor 3 that is coated on the upper surface of the fabric layer 2 from leaking out. The hydrogel precursor 3 comprises a mixture of first and second reagents. The first reagent contains a photo-initiator and a cross-linking agent. The second reagent contains acrylic acid-based monomers. The first and second reagents are individually prepared before mixing. The hydrogel precursor 3 can be applied by coating, e.g., spray coating, onto the upper surface of the fabric layer 1.

An example of the photo-initiator includes 1-hydroxyl cyclohexyl phenyl ketone. An example of the cross-linking agent includes poly(ethylene glycol) diacrylate. An example of the acrylic acid-based monomer includes DOUBLEMER® 013.

A portion of the applied hydrogel precursor 3 is then infiltrated into the fabric layer 2 such that the hydrogel precursor 3 is dispersed in the fabric layer 2. The hydrogel precursor 3 is then cured to form a hydrogel in and on the fabric layer 2. In an example, the hydrogel precursor 3 is cured using ultraviolet light. The solidification of the hydrogel precursor 3 leads to the complete assembly of a hydrogel laminate 10. The infiltrated hydrogel precursor 3 forms of an integrated part with the fabric layer 2 during the curing process. The coalescence between the hydrogel layer 3 and the fabric layer 2 allows a strong bond to form an unobvious boundary therebetween. The thickness of the integrated part depends on the depth in which the hydrogel precursor 3 has permeated into the fabric layer 2.

When manufacturing the hydrogel laminate 10 of the present invention, the film composite, which comprises the fluid-impermeable layer 1 and fabric layer 2, is unwound from a feed roller and a sheet thereof is fed onto a conveyor belt at a speed in the range of 0.3 to 30 meter/min, preferably, 3.2 meter/min. The film composite is delivered and advanced in such a manner that the upper surface of the fabric layer 2 faces up beneath a dispenser containing the hydrogel precursor 3. The hydrogel precursor 3 coated on the fabric layer 2 is evenly spread with a blade before curing. The film composite together with the hydrogel precursor 3 then enters an ultraviolet light chamber to allow the curing of the hydrogel precursor 3 to form a hydrogel layer 4 on the upper surface of the fabric layer 2. The resulting sheet comprising the fluid-impermeable barrier layer 1, the fabric layer 2 and the hydrogel layer 4 is the hydrogel laminate 10 of the current invention. A release film 7 is placed on top of the hydrogel laminate 10 before collecting using a collecting roller. The hydrogel laminate 10 produced in a form of a continuous strip can be tailored into desired sizes suitable for a wound dressing. This manufacturing method allows the hydrogel laminate 10 to be manufactured in a continuous process with low cost and high yield.

Preferably, ultraviolet light is an ultraviolet -A light having a wave length in a range of 315 to 400 nm with a peak value of 365 nm. The distance between ultraviolet light lamp and the film composite is 5 mm to 50 cm, preferably 25 cm. The exposure time of ultraviolet light is 2 second to 3 min, preferably, 0.3 minutes. The exposure energy is 3454.7 mj/cm².

The hydrogel laminate 10 of this invention is composed of the fluid-impermeable barrier layer 1, the hydrogel layer 4, and the fabric layer 2 sandwiched therebetween. In this structure, the hydrogel gel 4 and the fabric layer 2 are bonded to each other by virtue of the integrated part.

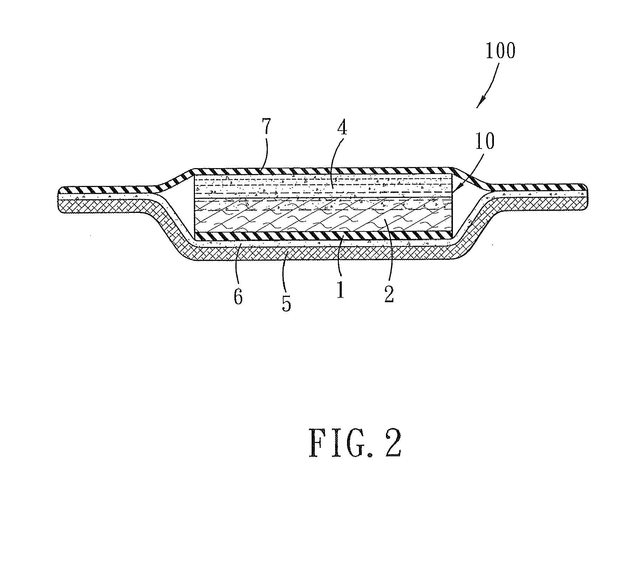

Referring to Figure 2, the hydrogel laminate 10 can be tailored into sizes appropriate for a wound dressing 100. The wound dressing 100 comprises : a vapor permeable film 5, the hydrogel laminate 10, an adhesive layer 6 disposed between the vapor permeable film 5 and partially covered by the hydrogel laminate 10, and a release film 7. The vapor permeable film 5 is made of a material that is water-resistant and vapor permeable. An example of such material is polyurethane. The fluid-impermeable barrier layer 1 of the hydrogel laminate 10 is adhered to the adhesive layer 6. Since the fluid-impermeable barrier layer 1 and the fabric layer 2 are respectively adhered to the adhesive layer 6 and the hydrogel layer 4, direct contact of the hydrogel layer 4 and the adhesive layer 6 can be avoided, thus preventing delamination of the hydrogel layer 4 and the adhesive layer 6 when large amount of exudate is absorbed, and maintaining structural integrity. Since the adhesive layer 6 is partially exposed from the hydrogel laminate 10, the exposed area of the adhesive layer 6 can adhere to the skin surrounding the wound. The release film 7 is used to cover the hydrogel laminate 10 and the exposed adhesive layer 6 to prevent contamination before usage, and can be peeled off when using the wound dressing 100.

To sum up, the method of the present invention provides a hydrogel laminate resistant to delamination due to the water-repellant surface formed between the fluid-impermeable barrier layer 1 and the fabric layer 2, in which a strong bond is formed therebetween. Delamination can be further prevented by the integrated part formed by the curing of the hydrogel precursor 3 that is infiltrated into the fabric layer 2. When the hydrogel laminate 10 of the present invention is used in the wound dressing 100, the structure and configuration thereof avoids direct contact of the hydrogel layer 4 and the adhesive layer 6, thereby preventing the occurrence of delamination and maintaining structural integrity of the wound dressing 100. Moreover, the method for manufacturing the hydrogel laminate 10 of the present invention is highly efficient due to the continuous production thereof, and eliminates the use of a mold.

## Claims

1. A method for manufacturing a hydrogel laminate (10) of a wound dressing (100), **characterized by** the steps of:
(a) providing a fabric layer (2);
(b) forming a fluid-impermeable barrier layer (1) on a lower surface of the fabric layer, the fluid-impermeable barrier layer (1) having a water-repellant surface bonded to the lower surface of the fabric layer (2);
(c) applying a hydrogel precursor (3) onto an upper surface of the fabric layer (2) so as to permit the hydrogel precursor (3) to infiltrate into the fabric layer (2); and
(d) curing the hydrogel precursor (3)in the fabric layer (2) to obtain a hydrogel laminate (10).

2. The method according to Claim 1, **characterized in that**, in step (b), the fluid-impermeable barrier layer (1) is formed by extrusion coating a molten thermoplastic material on the fabric layer (2).

3. The method according to Claim 2, **characterized in that,** in step (b), the thermoplastic material is selected from the group consisting of polyethylene, polypropylene, thermoplastic polyurethane, and combinations thereof.

4. The method according to Claim 1, **characterized in that** step (b) is implemented by heating or pressing a film of a plastic material and the fabric layer (2) so as to permit said film to be laminated with the fabric layer (2).

5. The method according to Claim 4, **characterized in that** the plastic material is selected from the group consisting of polyethylene, polypropylene, thermoplastic polyurethane, and combinations thereof.

6. The method according to Claim 1, **characterized in that** said fabric layer (2) is selected from the group consisting of woven, nonwoven, or knitted fabrics.

7. The method according to Claim 1, **characterized in that** the hydrogel precursor (3) is obtained by mixing a first reagent that contains a photo-initiator and a cross-linking agent, and a second reagent that contains acrylic acid-based monomers.

8. The method according to Claim 7, **characterized in that** said photo-initiator is 1-hydroxyl cyclohexyl phenyl ketone.

9. The method according to Claim 7, **characterized in that** said cross-linking agent includes poly(ethylene glycol)diacrylate.

10. The method according to Claim 1, **characterized in that** the hydrogel precursor (3) is cured using an ultraviolet light.

11. The method according to Claim 10, **characterized in that** the hydrogel precursor (3) is exposed to ultraviolet light for 2 seconds to 3 min.

12. A hydrogel laminate (10) for a wound dressing (100), **characterized by**:
a fabric layer (2);
a fluid-impermeable barrier layer (1) having a water-repellant surface bonded to a lower surface of said fabric layer (2); and
a cured hydrogel (4) in said fabric layer.

13. The hydrogel laminate (10) for a wound dressing according to Claim 12, **characterized in that** said fluid-impermeable barrier layer (1) is formed by extrusion coating a molten thermoplastic material on said fabric layer (2).

14. The hydrogel laminate (10) for a wound dressing according to Claim 12, **characterized in that** said fluid-impermeable barrier layer (1) is formed by heating or pressing a film of a plastic material layer upon said fabric layer (2) so as to permit said film to be laminated with said fabric layer (2).

15. A wound dressing (100), **characterized by**:
a vapor permeable film (5);
a hydrogel laminate (10) of claim 11;
an adhesive layer (6) disposed between said vapor permeable film (5) and said hydrogel laminate (10) and partially covered by said hydrogel laminate (10); and
a release film (7) covering said hydrogel laminate (10) and adhered to said adhesive layer (6) that is exposed from said hydrogel laminate (10).
